# EUROPÄISCHE PATENTSCHRIFT

(11) **EP 2 089 075 B1**
(45) Veröffentlichungstag und Bekanntmachung des Hinweises auf die Patenterteilung: **04.04.2012**
(21) Anmeldenummer: 07820768.5
(22) Anmeldetag: 01.10.2007
(51) Int. Cl.: A61L 27/56, A61L 27/58, A61L 27/48

(54) **MATRIX-GEL-TRANSPLANTAT OHNE ZELLEN**
MATRIX GEL GRAFT WITHOUT CELLS
GREFFON ACELLULAIRE À GEL MATRICIEL

(30) Priorität: 06.10.2006 DE 102006047346
(43) Veröffentlichungstag der Anmeldung: 19.08.2009
(73) Patentinhaber: BioTissue AG, 8038 Zürich (CH)
(72) Erfinder: KAPS, Christian, 10965 Berlin (DE); TÁNCZOS, Eszter, CH-8700 Kuesnacht (CH)
(74) Vertreter: Westendorp | Sommer
(86) Internationale Anmeldenummer: PCT/EP2007/060384
(87) Internationale Veröffentlichungsnummer: WO 2008/040702

(56) Entgegenhaltungen:
- EP-A- 1 273 312
- WO-A-01/54735
- WO-A-2006/104901
- DE-A1-102005 030 614
- US-A1- 2002 119 179

## Beschreibung

Die vorliegende Erfindung betrifft ein zellfreies Matrix-Gel-Transplantat zur Gewebsregeneration und insbesondere zur Knorpelregeneration, ein Verfahren zu dessen Herstellung und die Verwendung des Transplantats zur Gewebsregeneration.

### Stand der Technik

Für die normale Gelenkfunktion ist der Gelenkknorpel als Gleitfläche unerläßlich. Schädigungen des Gelenkknorpels treten z.B. bei Osteoarthrose, Trauma oder Osteochondrosis dissecans auf. Die Gelenkknorpelzellen, welche den Gelenkknorpel aufbauen, weisen bei Erwachsenen nur eine geringe Regenerationsfähigkeit auf.

Der Gelenkknorpel ist eine vom Bindegewebe abgeleitete mesodermale Gewebsform, welche auf multipotente, undifferenzierte mesenchymale Vorläuferzellen zurückzuführen ist. Der hyaline Knorpel ist die am weitesten verbreitete Knorpelform und findet sich beispielsweise in den Gelenkflächen. Knorpeldefekte aufgrund von Abnutzung oder Beschädigung stellen ein weit verbreitetes medizinisches Problem dar. Daher wurden schon in der Vergangenheit, vor allem aber in den letzten Jahren Methoden und Techniken entwickelt, um defekte chondrale oder auch osteochondrale Areale im Gelenkknorpel zu ersetzen. So wurden als Gelenkknorpelersatz Periost-, Perichondrium-, allogene und autologe osteochondrale Transplantate, allogene Menisken oder auch Prothesen aus künstlichen Materialien eingesetzt.

Bei der autologen Transplantation von Chondrozyten werden dem Patienten entnommene Chondrozyten in Zellkultur vermehrt und dem Patienten wieder zurückgegeben. Die Rückführung kann in Form verschiedenster Transplantate erfolgen. Beispiele hierfür sind Injektionslösungen, die in das Gelenk injiziert werden, mit Knorpelzellen beimpfte Matrices und Ähnliches.

Beispielsweise beschreibt die WO 97/15655 künstliche Gewebe, die aus dreidimensionalen extrazellulären Matrices und gentechnisch manipulierten Zellen bestehen, wobei die Matrices immunsuppressive oder zelldifferenzierende Faktoren freisetzen können. Bevorzugt handelt es sich um Matrices in Form eines Polymervlieses, in die eine Zellsuspension, die beispielsweise in einer Fibrinogenlösung suspendiert sein kann, verteilt wird. Der Matrix können zudem Faktoren oder Komponenten der entsprechenden extrazellulären Matrix zugesetzt werden, die dem Wachstums- und/oder Differenzierungsprozess förderlich sind. Um die Zellen in der Matrix zu halten, kann die Zellsuspension durch Thrombinzugabe verfestigt werden, um das fertige Transplantat zu erhalten.

Die DE 44 31 598 beschreibt ein Verfahren zum Herstellen eines Implantates aus Zellkulturen, bei dem dreidimensionale Trägerstrukturen, an die Zellen angelagert sind, zunächst ummantelt und anschließend mit einer Nährlösung perfundiert werden. In die Trägerstrukturen sind resorbierbare Mikrokörper eingearbeitet, die bei der Resorption die Gewebebildung beeinflussende Faktoren freigeben.

Die DE 100 06 822 beschreibt ein Verfahren zum Herstellen eines Knochen- oder Knorpeltransplantates, bei dem bioresorbierbare und biokompatible Gerüststrukturen aus durch Fibrin oder Hydrogel vernetzte osteogene Zellen und Faktoren bestehen und zu aneinandersetzbaren geometrischen Körpern geformt worden sind.

Die DE 43 06 661 beschreibt eine dreidimensionale Trägerstruktur, vorzugsweise aus einem Polymervlies, in die Zellen eingelagert werden. Die Trägerstruktur wird anschließend in Nährlösung perfundiert, um das Zellwachstum und die Ausbildung einer extrazellulären Matrix durch die Zellen zu fördern. Um ein Auswandern bzw. Ausspülen der Zellen zu verhindern, wird die Trägerstruktur mit Agarose ummantelt.

Aus der DE 101 39 783 ist ferner bekannt die Bereitstellung von mesenchymalen Zellen in Synovialflüssigkeit. Diese Zusammensetzung kann, falls gewünscht, auch auf einen Träger wie ein Vlies oder einen Kunststoff aufgebracht und in dieser Form als Transplantat verwendet werden. Ansonsten wird die Suspension der Zellen in Synovialflüssigkeit als solches in das betroffene Gelenk injiziert.

Alternativ werden Matrixstrukturen synthetisiert, welche selbst keine Zellen enthalten. So beschreibt beispielsweise die US 2003/0003153 versteifte Matrixmembranen, die ein oder mehrere gerüstbildende Proteine enthalten, welche für das Zellwachstum geeignet sind. In diesen Fällen wird davon ausgegangen, dass Zellen aus körpereigenem Gewebe in die Matrixstruktur einwandern. Dieses wird beispielsweise mittels der konventionellen Pridie-Bohrung oder Mikrofrakturierung erzielt. Bei diesen Techniken werden geringfügige Bohrungen oder Frakturen in den Gelenkknochen bis auf das Knochenmark eingebracht. Durch die Bohrungen kommt es zur Einblutung in den Defekt, wodurch sich der Defekt mit einem Blutpfropf füllt. In dem Pfropf befinden sich mesenchymale Vorläuferzellen, welche durch entsprechende Reize stimuliert ein knorpelartiges Ersatzgewebe, den sog. Faserknorpel bilden können. Wird über der Pridie-Bohrung ein Matrixmaterial bereitgestellt, können die Blutzellen in dieses Matrixmaterial einwandern und sich dort ansiedeln.

Die DE 199 57 388 und WO 2005/014027 nutzen diesen Effekt und verstärken ihn, indem in der Matrixstruktur Wachstums- und Differenzierungsfaktoren (DE 199 57 388), Chemokine (WO 2005/014027) bzw. Blutserum (DE 10 2005 030 614) als Rekrutierungsmittel bereitgestellt werden. Alle Faktoren sollen zur verstärkten Rekrutierung der knorpelbildenden mesenchymalen Vorläuferzellen führten, wodurch letztlich eine schnellere Regeneration des Knorpels erzielt werden soll.

Aus der WO 02/00272 ist schließlich die Möglichkeit bekannt, entsprechende Transplantate auch aus Blut und einer Polymerkomponente herzustellen. Diesem Dokument liegt das Problem zugrunde, dass der bei der Pridie-Bohrung üblicherweise entstehende Blutpfropf sich bei der Gerinnung zusammenzieht und damit die Form verändert. Das zugesetzte Polymer verhindert diese Formveränderung und gestattet somit eine formtreue Verheilung. Zur Herstellung des Transplantats wird ein Polymer mit Blut oder einer Blutkomponente wie Erythrocyten, Leukozyten, Monozyten, Plättchen, Fibrinogen, Thrombin und plättchenreichem Plasma gemischt und in den Defekt eingebracht. Bei der Verwendung einer Blutkomponente ist jedoch das Vorhandensein gerinnungsfähigen Materials wesentlicher Bestandteil, um die gewünschte Wirkung zu erzielen.

EP 1 273 312 A2 offenbart ein Implantat zur Knorpelgeweberegeneration, das Chondrozyten oder deren Progenitorzellen, sowie ein Gerüst umfasst. Dieses Gerüst umfasst ein Kompositmaterial, das ein Netz oder einen porösen Schwamm aus biologisch abbaubaren synthetischem Polymer und einen porösen Schwamm eines Natur-abgeleiteten Polymers umfasst, der auf und/oder in dem Netz oder porösen Schwamm ausgebildet ist.

WO 2006/104901 A2 beschreibt ein Gerüst, das eine dreidimensionale poröse Matrix aus Polycaprolacton aufweist, die optional mit mindestens einem ausgewählten extrazellulären Material beschichtet ist.

WO 01/54735 A2 lehrt eine Gel-infundierte Schwamm-Matrix, sowie ein Verfahren zur Herstellung eines Gel-infundierten Matrixmaterials. Dieses Verfahren umfasst, dass ein Gel mit bioaktiven Agentien in ein poröses biologisch abbaubares Matrixmaterial einziehen gelassen wird.

US 2002/0119179 A1 offenbart eine implantierbare, biologisch abbaubare Vorrichtung, die eine Faser-Matrix enthält, wobei die Faser-Matrix aus Fasern A und Fasern B hergestellt ist. Hierbei sind die Fasern A schneller biologisch abbaubar als die Fasern B. Zudem liegen die Fasern A und B in ausgewählten relativen Mengen vor.

Aus der US2005/0043814 ist schließlich ein zellfreies Matriximplantat mit optionaler knocheninduzierender Zusammensetzung bekannt, welches ein kollagenes, thermoreversibles Gel, eine aromatische organische Säure oder eine adsorbierbare Caprolactonpolymer-Trägermatrix umfaßt. Die knocheninduzierende Zusammensetzung kann aus Polyglykolsäurepolymeren bestehen und auf eine Matrix aus Kollagenen oder Polyglykolsäuren aufgebracht werden. Bei Verwendung dieses zellfreien Matriximplantates ist die Thermoreversibilität des Gels ein wesentlicher Bestandteil, da dadurch, die Zusammensetzung in flüssiger Form appliziert werden kann. Erst nach Injektion der flüssigen Zusammensetzung härtet diese im Körper des Patienten aus.

Bei den oben beschriebenen Technologien bestehen Nachteile dahingehend, dass dann, wenn das Transplantat selbst Zellen enthält, diese häufig durch die Manipulation bei der Handhabung beschädigt werden, das Transplantat bei Verwendung von Zellen, insbesondere autologer Zellen über ein langwieriges Kulturverfahren hergestellt werden muss und die sorgfältige Kontrolle auf Kontaminationen erfordert und schließlich keine Lagerfähigkeit, bzw. nur eine Lagerung unter aufwändigen Bedingungen, besteht.

Diese Nachteile werden bei der Verwendung von allogenen, körperfremden Zellen noch dahingehend verstärkt, daß eine aufwendige bakteriologische und virologische Untersuchung der Spenderzellen notwendig ist, um die Übertragung von Krankheiten durch das zellhaltige Transplantat zu vermeiden. Zudem ist die Gefahr einer Abstoßungsreaktion bei der Verwendung von körperfremden Zellen gegeben.

Weitere Nachteile der oben beschriebenen Technologien bestehen darin, daß zellfreien Transplantaten Faktoren zugesetzt werden, die die Einwanderung von Zellen beschleunigen bzw. ermöglichen. Diese Faktoren können beispielsweise entweder Wachstums- und Differenzierungsfaktoren oder Chemokine sein. Diese Faktoren sind tierischen Ursprungs, d.h. aus Tieren isoliert oder werden rekombinant mittels Bakterien oder Hefen hergestellt. Bei der rekombinanten Herstellung werden jedoch überwiegend Faktoren produziert, die auf eine Struktur tierischen Ursprungs zurückgehen. Dieses ist nachteilig, da es aufgrund des Unterschiedes zwischen "Spender" und "Empfänger" dieser Bestandteile des Transplantats nach der Transplantation leicht zu Unverträglichkeiten oder allergischen Reaktionen kommen kann.

Die Verwendung von Blut, Blutkomponenten oder Serum zur Rekrutierung von Zellen in das zellfreie Transplantat ist in so fern unzureichend, da bei der Verwendung von allogenen, körperfremden Blut ebenfalls aufwendige bakteriologische und virologische Untersuchungen zur Vermeidung der Übertragung von Krankheiten notwendig sind. Die Verwendung von körpereigenem Blut, Blutkomponenten oder Serum setzt hingegen eine zusätzliche Manipulation am Transplanat (Einbringen der Komponente) und am Patienten (Blutabnahme) voraus. Jede Manipulation am Transplantat birgt die Kontaminationsgefahr des Transplantates in sich, was ebenfalls zu Unverträglichkeiten beim Patienten führen kann. Zudem ist die dafür notwendige zusätzliche Entnahme von Patientenmaterial mit einem hohen Zeitaufwand und zusätzlichen Kosten verbunden, welche nicht wünschenswert sind.

Bei der Verwendung von Implantaten, die sich nach der Implantation verfestigen, besteht der Nachteil, dass ein festes, ausgehärtetes Implantat das umliegende Gewebe geringerer Festigkeit/Härte mechanisch schädigt. Zusätzlich behindert ein festes Implantat das Einwandern von Zellen und benötigt sehr lange Abbauzeiten bzw. Resorbtionszeiten.

Ein weiterer Nachteil der oben beschriebenen zellfreien Transplantate ist, daß diese im Stand der Technik nach einer Pridie-Bohrung oder Mikrofrakturierung eingesetzt werden, um sämtliche beim Einbluten mit eingebrachten bzw. eingeschwemmten Zellen im zellfreien Transplantat unselektiv zu beherbergen. Dadurch kann es zu einer Überwucherung des Transplantats mit Gewebeuntypischen Zellen und/oder Bestandteilen kommen, welche die Ausbildung des gewünschten Gewebes behindern könnte oder die Ausbildung eines Mischgewebes fördert. Solch eine Einblutung in den Defekt ist insofern nachteilig, da es zu Irritationen und Entzündungen des umliegenden Gewebes führen kann. So beschreiben Hooiveld und Kollegen, daß das Zusammenbringen von Knorpel mit Blut oder Blutkomponenten für 4 Tage zu einer Schädigung der Knorpelzellen führt [Hooiveld M.J. et al.: Haemoglobin-derived iron-dependent hydroxyl radical formation in blood-induced joint damage: an in vitro study, Rheumatology 43, 784-790, 2003]. Hooiveld beschreibt weiterhin, daß eine Einblutung in das Gelenk zu einer Gelenkinnenhautentzündung (Synovitis) führen kann [Hooiveld M.J. et al.: Immature articular cartilage is more susceptible to blood-induced damage than mature articular cartilage: an in vivo animal study, Arthritis Rheum 48, 396-403, 2003].

Besonders nachteilig bei den aus dem Stand der Technik bekannten Transplantaten ist, dass diese aufgrund der enthaltenen Zellen oder biologischen Bestandteile nur sehr begrenzt lagerbar sind und zusätzlich sehr spezifische Lagerbedingungen benötigen.

Die vorliegende Erfindung hat unter Anderem die Aufgabe, ein Transplantat bereitzustellen, welches einfach herzustellen ist, möglichst wenig Manipulationsschritte zur Herstellung benötigt und sehr gut zu lagern ist. Darüber hinaus sollte es schnell und einfach anwendbar sein, aber trotzdem vergleichbare und/oder zumindest genauso gute Therapieerfolge gewährleisten wie die im Stand der Technik bekannten Transplantate. Des Weiteren wäre es wünschenswert, möglichst auf die Verwendung von körperfremden, ggf. sogar rekombinanten Wachstumsfaktoren, welche potentiell Allergene darstellen, verzichten zu können. Auch wäre es wünschenswert, möglichst auf eine zusätzliche Blutentnahme oder die Verwendung von Blut, Blutkomponenten oder Serum verzichten zu können, da hierdurch eine zusätzliche Kontaminationsgefahr und Belastung des Patienten gegeben ist. Des Weiteren wäre es wünschenswert, möglichst die Einblutung in den Defekt nach einer Pridie-Bohrung oder Mikrofrakturierung verhindern zu können, um einer Schädigung des umliegenden Gelenkgewebes vorbeugen zu können. Auch wäre es wünschenswert, daß das zellfreie Transplantat die Festigkeit bzw. Elastizität des umliegenden Gewebes aufweist, um eine mechanische Schädigung des umliegenden Gewebes zu verhindern. Wünschenswert wäre in diesem Zusammenhang eine mögliche Adaption der Härte/Elastizität des Transplantats an den individuellen Patienten um unharmonische Bewegungen bedingt durch verschiedene Materialhärten zu minimieren.

### Zusammenfassung der Erfindung

Die vorliegende Erfindung löst die Probleme des Standes der Technik. Hierzu stellt sie ein zellfreies Matrix-Gel-Transplantat bereit, bestehend aus
(i) einer zusammenhängenden, gerüstbildenden Matrix mit offener Porosität aus einem biologisch und pharmazeutisch annehmbaren Material, wobei die Matrix ein Material umfasst, ausgewählt aus der Gruppe, bestehend aus Polyglycolsäure, Polymilchsäure, Poly(glycolid,lactat) und Gemischen davon, und
(ii) einem Gel aus einem biologisch und pharmazeutisch annehmbaren Material, wobei das Gel auf mindestens einer Seite der Matrix aufgebracht ist und/oder diese mindestens teilweise durchdringt und das Gel Hyaluronsäuregel ist.

In einem zweiten Aspekt wird ein Verfahren zur Herstellung eines solchen zellfreien Matrix-Gel-Transplantats bereitgestellt, umfassend die folgenden Schritte:
(v) In-Kontakt-bringen der Matrix mit dem Gel, wobei das Gel auf mindestens einer Seite der Matrix aufgebracht ist und/oder diese mindestens teilweise durchdringt und das Gel Hyaluronsäuregel ist, und wobei die Matrix ein Material umfasst, ausgewählt aus der Gruppe, bestehend aus Polyglycolsäure, Polymilchsäure, Poly(glycolid,lactat) und Gemischen davon, und
(vi) Trocknen des in (v) gebildeten Matrix-Gel-Komplexes.

In einem dritten Aspekt stellt die vorliegende Erfindung schließlich ein zellfreiers Matrix-Gel-Transplantat zur Verwendung zur Abdeckung und Erhöhung der Viskoelastizität von Defekten zur Gewebsregeneration und insbesondere zur Regeneration mesenchymaler Gewebe, insbesondere von Knorpel und/oder Knochen bereit.

### Kurze Beschreibung der Figuren

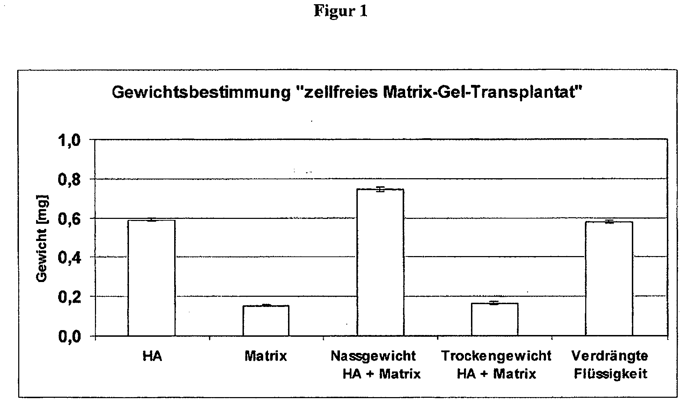

Fig. 1 zeigt das jeweilige Gewicht eines zellfreien Transplantats vor und nach der Trocknung mittels Lyophilisation sowie das Gewicht der verdrängten Flüssigkeit. Hierbei bedeuteten die Abkürzungen "HA" Hyaluronsäure und "mg" Milligramm. Die genaue Versuchsanordnung, die dieser Figur zugrunde liegt, ist in Beispiel 1 beschrieben.

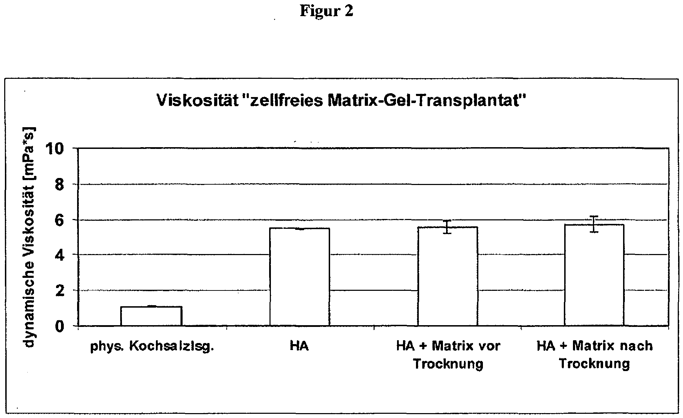

Fig. 2 zeigt die dynamische Viskosität von Hyaluronsäure und zellfreien Transplantaten vor und nach der Trocknung mittels Lyophilisation. Die Abkürzung "phys. Kochsalzlsg" bedeutet physiologische Kochsalzlösung, "HA" bedeutet Hyaluronsäure und "mPa*s" ist die Einheit der dynamischen Viskosität in Millipascal mal Sekunde. Die genaue Versuchsanordnung, die dieser Figur zugrunde liegt ist in Beispiel 2, beschrieben.

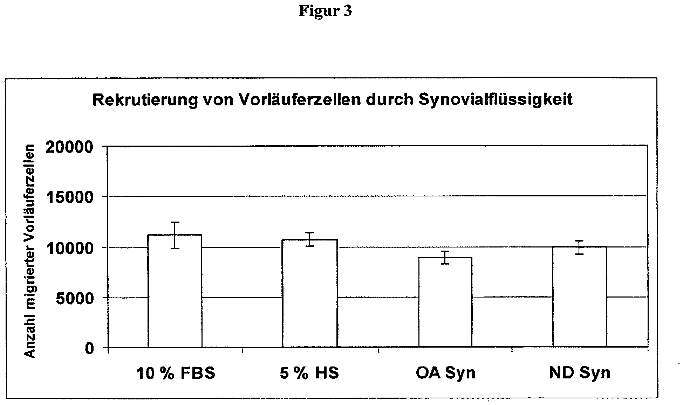

Fig. 3 zeigt die chemotaktische Wirkung (Rekrutierung) von humaner Synovialflüssigkeit auf humane mesenchymale Stammzellen *in vitro.* Die Abkürzung "FBS" bedeutet fötales Rinderserum (fetal bovine serum), "HS" steht für humanes Serum, "OA Syn" ist Synovialflüssigkeit von Patienten mit Osteoarthrose und "ND Syn" ist Synovialflüssigkeit von gesunden (normal donor) Spendern. Die genauen Daten, die dieser Figur zugrunde liegen sind in Beispiel 4 beschrieben.

### Genaue Beschreibung der Erfindung

Die vorliegende Erfindung betrifft, ein zellfreies Implantat, bestehend aus (i) einer zusammenhängenden, gerüstbildenden Matrix mit offener Porosität aus einem biologisch und pharmazeutisch annehmbaren Material wobei die Matrix ein Material umfasst, ausgewählt aus der Gruppe, bestehend aus Polyglycolsäure, Polymilchsäure, Poly(glycolid,lactat) und Gemischen davon, und (ii) einem Gel aus einem biologisch und pharmazeutisch annehmbaren Material, wobei das Gel auf mindestens einer Seite der Matrix aufgebracht ist und/oder diese mindestens teilweise durchdringt und das Gel Hyaluronsäuregel ist.

Die Matrix des erfindungsgemäßen zellfreien Transplantats ist eine zusammenhängende, gerüstbildende Matrix mit offener Porosität. Mit dem Ausdruck "zusammenhängend" ist hierin gemeint, dass die Matrix eine Handhabung des Transplantates gestattet, ohne hierbei in Einzelteile oder Bestandteile zu zerfallen. Nicht erforderlich ist, dass alle Bestandteile der Matrix über chemische Bindungen oder Wechselwirkungen aneinander gebunden sind. Eine mechanische Verbindung durch beispielsweise Weben, Walken, Verdrillen oder Ähnliches ist ausreichend.

Mit dem Ausdruck "gerüstbildend" ist hiermit die Eigenschaft der Matrix gemeint, als Strukturbildner für die von den eingewanderten Zellen zu erstellende Gewebematrix zu fungieren. Die Matrix bildet zudem ein Gedüst oder Gatter, in dem sich die Zellen ansiedeln können und Halt finden, um nicht z.B. von Synovialfluid oder Blut aus der Matrix ausgeschwemmt zu werden.

Mit "offener Porosität" ist schließlich im Sinne der Erfindung gemeint, dass die Zwischenräume zwischen den Gerüststrukturen der Matrix einem Stoff- und insbesondere Fluidaustausch mit der Umgebung der Matrix zugänglich sind. Vorzugsweise ist die Porengröße der Poren so bemessen, dass auch ein Eindringen bzw. ein Spülen von Zellen ermöglicht wird. Unter offener Porosität wird im Sinne der Erfindung jedoch auch eine Struktur verstanden, wie sie in Gelen vorliegt. Hier werden durch das Skelett des Gelbildners die Gerüststrukturen der Matrix bereitgestellt. Zwischen diesen befinden sich Hydrathüllen und Fluid, in welche ein Eindringen der Zellen und mit welchen ein Fluidaustausch möglich ist. Entsprechende Gelstrukturen werden daher auch als Matrices mit offener Porosität im Sinne der vorliegenden Erfindung verstanden.

Die Gerüststrukturen mit offener Porosität sind vorzugsweise ausgewählt unter gewebten oder nicht gewebten Geweben (Gewirke) insbesondere Vlies- und Filzstrukturen, Membranen, Schwämmen, Watte, offenzelligen Schäumen, Wolle, Flechtwerk, geordneten und ungeordneten Faserbündeln, porösen Keramikmaterialien, Spongiosa und Gelen sowie Kombinationen derselben. Bevorzugt weist die Matrix eine Vlies- oder Filzstruktur auf. Kombinationen verschiedener Strukturen beispielsweise in lagenförmiger Anordnung sind möglich und im Bereich der vorliegenden Erfindung.

Das Matrixmaterial ist eingeeignetes, biologisch und pharmazeutisch annehmbares Material. Das Matrixmaterial, das in der erfindungsgemäBen Matrix verwendet wird, kann resorbierbar oder nicht resorbierbar sein. Resorbierbare Materialien sind bevorzugt. Die Matrix umfasst ein Material, ausgewählt aus der Gruppe, bestehend aus Polyglycolsäure, Polymilchsäure, Poly(glycolid, lactat) und Gemischen davon. Ganz besonders bevorzugt handelt es sich um Polyglycolsäure (PGA) oder Polymilchsäure.

Als Polyglycolsäuren werden vorzugsweise reine Polyglycolsäuren mit Molekulargewichten von > 20.000, vorzugsweise 30.000 bis 70.000 g/mol, am meisten bevorzugt ca. 50.000 g/mol verwendet. Als Matrixmaterial kann beispielsweise ein Vlies aus Polyglycolsäure, wie es von der Alpha Research Switzerland GmbH unter der Marke PGA-Soft Felt® vertrieben wird, verwendet werden. Dieses Material ist CE-zertifiziert und daher für Arzneimittelzwecke geeignet. Bei diesem Produkt beträgt die Resorptionszeit *in vivo* ca. 40 bis 60 Tage. Nach sieben Tagen *in vitro* beträgt die mechanische Festigkeit in Folge der Hydrolyse noch ca. 50 % des Ausgangswertes.

Das erfindungsmäße zellfreie Transplantat umfaßt neben der Matrix ein Gel. Dieses Gel ist auf mindestens einer Seite der Matrix aufgebracht und/oder durchdringt diese mindestens teilweise. Bevorzugt durchdringt das Gel die Matrix vollständig. Die Matrix selbst weist vorzugsweise eine andere Struktur als die eines Gels auf. Ganz besonders bevorzugt sind steifere Strukturen wie sie oben mit Ausnahme der Gele explizit genannt sind. Das Gel hat demnach vorzugsweise eine geringere Steifigkeit als die Matrix. Am meisten bevorzugt sind Vlies- und Filzstrukturen, in die ein Gel eingebracht ist.

Das Gel kann ein natürliches oder synthetisches Hydrogel sein. Es weist vorzugsweise eine geringere Steifigkeit als die Matrix auf. Das Gel kann Hyaluronsäure sein. Geeignete Salze sind z.B. Alkali- und Erdalkalisalze der genannten Gele. Am meisten bevorzugt handelt es sich um Hyaluronsäure oder ein Hyaluronsäurederivat, insbesondere Hyaluronsäuressalz wie Na-Hyaluronat. Wie in Fig. 1 dargestellt, zeigt insbesondere die Verwendung von Hyaluronsäure in Kombination mit einer erfindungsgemäßen Matrix besonders vorteilhafte Verhältnisse von Nassgewicht und Trockengewicht und ist deshalb in der Verarbeitung des Transplantates, der Trocknung und/oder Lagerung besonders geeignet.

Durch Zugabe von einer bestimmten Menge einer physiologisch geeigneten Lösung kann die Härte/Festigkeit des Transplantats der Knorpel- und/oder Knochenhärte sowie dem Gewebe des Patienten angepasst werden.

Als Hyaluronsiurequalitäten können fermentativ hergestellte Qualitäten verwendet werden. Alternativ ist auch die Verwendung von tierisch gewonnener Hyaluronsäure möglich. Das mittlere Molekulargewicht der verwendeten Qualitäten beträgt üblicherweise zwischen 250 und 6.000 kDa, vorzugsweise 1.000 bis 2.000 kDa, am meisten bevorzugt in etwa 1.200 kDa. Geeignete Hyaluronsäureprodukte sind im Handel erhältlich. Geeignet ist beispielsweise die von der TRB Chemedika AG unter der Marke Ostenil® vertriebene Hyaluronsäurequalität. Dieses Material ist CE-zertifiziert und daher für Arzneimittelzwecke geeignet.

Die Gele können durch Quellen, Präzipitation oder Polymerisation eines geeigneten Gelbildners in einer physiologisch geeigneten Lösung gebildet werden. Beispiele für derart geeignete Lösungen sind Wasser sowie wässrige Lösungen von Salzen (z.B. Alkali- und Erdalkalihalogeniden (Cl, Br, I), -carbonaten, -phosphaten, -citraten -acetaten und Ähnlichem), organischen Säuren, Puffersubstanzen und deren Gemischen. Alternativ können komplexere Lösungen verwendet werden wie Kulturmedium oder Körperfluide oder hiervon abgeleitete Lösungen wie Synovialfluid. Die verwendete Menge an Gelbildner ist so bemessen, dass sie eine angemessene Viskosität des Gels bereitstellt. Für Hyaluronsäure liegt diese üblicherweise im Bereich von 0,5-50 mg/ml, vorzugsweise 0,5-20 mg/ml, am meisten bevorzugt 10 mg/ml.

Am meisten bevorzugt ist ein Transplantat aus einem Polyglycolsäure-(PGA) Vlies- oder Filz als Matrix, in die ein Hyaluronsäuregel eingearbeitet ist.

Die Abmessungen des erfindungsgemäßen zellfreien Transplantats richten sich generell nach den Abmessungen des zu behandelnden Defekts bzw. der benötigten Größe des Transplantats. Die Dimensionen sind vom behandelnden Arzt nach Bedarf anzupassen. Für Läsionen in Knorpelgewebe, insbesondere im Kniegelenk liegen diese Größen üblicherweise im Bereich von 10 bis 50 mm Länge, 10 bis 50 mm Breite und 0,5 bis 3 mm Dicke, bevorzugt 10 bis 30 mm Länge, 10 bis 30 mm Breite und 1 bis 2 mm Dicke. Am meisten bevorzugt wären Größen von 20 x 30 mm Breite und Länge und 1,1 bis 2 mm Dicke. Entsprechende Abmessungen sind für nicht quadratische Formen z.B. rechteckig, rund, oval, polyedrisch etc. anpassbar.

Die Matrix kann nach dem In-Kontakt-Bringen mit dem Gel getrocknet werden. Das Trocknen des erfindungsgemäßen Implantates gestattet zum Einen die langfristige Lagerung und zum Anderen die einfache Verwendung des Implantes an sich. So kann das Implantat nach der Lagerung direkt in trockenem Zustand verwendet werden oder wieder mit einer wäßrigen Lösung In-Kontakt gebracht werden.

Das getrocknete Implant ermöglicht das einfache Einbringen von wäßrigen Lösungen vor der Verwendung des Implantates durch einen "Schwammeffekt". Die wäßrige Lösung wird durch einfaches Aufbringen auf das Implantat oder durch Einlegen des Implantates in die wäßrige Lösung in das Implantat gesogen. Bevorzugt für das Einbringen einer wäßrigen Lösung in das Implantat vor der Anwendung ist physiologische Kochsalzlösung und/oder synoviale Flüssigkeit.

Geeignete Konzentrationen der physiologischen Kochsalzlösung und/oder synovialen Flüssigkeit sind 1 bis 100 Vol.-% des von der Matrix gehaltenen Volumens an Gel und Fluid. Bevorzugt sind Konzentrationen von 10 bis 90 %, vorzugsweise 40 bis 70 % und am meisten bevorzugt 50 % des durch u.a. Kapillarkräfte gehaltenen Flüssigkeitsvolumens. Zur Verringerung der Konzentration von synovialer Flüssigkeit unter 100 % kann mit wäßriger Lösung verdünnte Synovialflüsigkeit eingesetzt werden. Bevorzugt wird die synoviale Flüssigkeit mit physiologischer Kochsalzlösung verdünnt.

Die Verwendung des erfindungsgemäßen, ungetrockneten oder getrockneten zellfreien Implantats ohne vorheriges In-Kontakt-Bringen mit einer wäßrigen Lösung zur Implantation in einen Defekt ermöglicht durch den vorliegenden Konzentrationsgradienten im Patienten das passive Eindringen von körpereigenen Flüssigkeiten - wie synovialer Flüssigkeit- in das Implantat. Die so in das Implantat eingedrungene synoviale Flüssigkeit mit eventuell enthaltenen Botenstoffe in wäßriger Lösung erhöht die Effizienz des Implantates zur Rekrutierung von mesenchymalen Vorläuferzellen aus dem Knochenmark in das Implantat bzw. den Defektort.

Die Verwendung des erfindungsgemäßen zellfreien ungetrockneten oder getrockneten Implantates nach dem In-Kontakt-Bringen mit physiologischer Kochsalzlösung vor der Implantation zur Implantation in einen Defekt ermöglicht die Ausbildung eines Konzentrationsgradienten an Botenstoffen/körpereigenen Stoffen in wäßriger Lösung, wie Wachstums- und Differenzierungsfaktoren und/oder Chemokine. Hierdurch werden passiv körpereigene Botenstoffe aus der synovialen Flüssigkeit über Diffusion in das zellfreie Implantat eingebracht, die die Effizienz des Implantates zur Rekrutierung von mesenchymalen Vorläuferzellen aus dem Knochenmark erhöhen.

Die chemotaktische Wirkung von Wachstums- und Differenzierungsfaktoren, wie beispielsweise "cartilage derived morhogenetic protein-1" (CDMP1) oder "growth and differentiation factor-5" (GDF5) und "cartilage derived morphogenetic protein-2" (CDMP2) oder "growth and differentiation factor-6" (GDF6) auf mesenchymale Stamm- und Vorläuferzellen sind in der DE 199 57 388 beschrieben. Die chemotaktische Wirkung bzw. die Verwendung von Chemokinen, wie beispielsweise "stromal derived factor-1α" (SDF1-α) oder Interleukin-8 (IL8), zur Rekrutierung von mesenchymalen Stamm- und Vorläuferzellen ist ebenfalls in der DE 103 33 901 beschrieben. Die chemotaktische Wirkung bzw. die Verwendung von humanem Serum zur Rekrutierung von Vorläuferzellen aus dem Knochenmark ist in der DE 10 2005 030 614 offenbart. Das Testverfahren zur Bestimmung der chemotaktischen Aktivität von Stoffen ist ebenfalls aus der DE 10 2005 030 614 bekannt.

Die Testung der chemotaktischen Wirkung von Synovialflüssigkeit von Normalspendern und Spendern mit Osteoarthrose auf mesenchymale Vorläuferzellen des Knochenmarks im in Fig. 3 beschriebenen Testverfahren ergab überraschenderweise, daß humane Synovialflüssigkeit von gesunden Spendern und von Spendern mit Osteoarthrose im Vergleich zu Serum eine vergleichbare Zahl von Vorläuferzellen rekrutiert. Die Zellzahlen der im Mittel mit entsprechenden Standardabweichungen rekrutierten mesenchymalen Vorläuferzellen sind in Figur 3 dargestellt. Die Ergebnisse sind in Beispiel 4 zusammengestellt.

Die Verwendung von synovialer Flüssigkeit im erfindungsgemäßen zellfreien Implantat gestattet überraschenderweise eine Erhöhung der Rekrutierungseffizienz von mesenchymalen Vorläuferzellen aus dem durchbluteten Knochenmark im Vergleich zu Wachstums- und Differenzierungsfaktoren und/oder Chemokinen um mehrere Größenordnungen (siehe Figur 3). Diese überraschend gesteigerte Rekrutierungseffizienz erlaubt es, auf die separate Einbringung von ausdifferenzierten Zellen oder Vorläuferzellen im Transplantat selbst zu verzichten. Dadurch wird die Handhabung des Transplantats enorm erleichtert, die Herstellung des Transplantats vereinfacht, da keine Manipulationsschritte am Transplantat nötig werden. Dadurch wird dessen Herstellungszeit sehr verkürzt, die Herstellung kostengünstig bei Vergleichsweiser bzw. genauso guter Rekrutierungseffizienz.

Überraschenderweise hat sich gezeigt, daß die Rekrutierungseffizienz von synovialer Flüssigkeit der Rekrutierungseffizienz von Serum des Blutes entspricht. Synoviale Flüssigkeit ist ein integraler Bestandteil des Gelenks und ist auf herkömmlichem Wege in einfacher Weise zu gewinnen. Dieses kann vorzugsweise, im Falle des In-Kontakt-Bringen vor der Implantation, direkt während der Implantation vom Patienten selbst erfolgen. Dem Patienten kann damit autologes Material re-implantiert werden, während ein Zusatz von anderen potentiell allergenen und/oder immunaktiven Faktoren nicht erforderlich ist. Ein zweiter Eingriff zur Entnahme von Blut des Patienten zur Serumgewinnung wird vermieden.

Da die Einwanderung der Zellen und/oder Faktoren in das Transplantat ohne die Verwendung von körperfremden Zellen oder dem Einsatz von körperfremden biologischen Botenstoffen ermöglicht wird, ist das Infektions- und allergene Risiko für den Patienten stark minimiert. Darüber hinaus kann dieses "vereinfachte" erfindungsgemäße Transplantat sehr gut getrocknet und/oder gelagert werden. Hierdurch ist es besonders kostengünstig und anwenderfreundlich.

Die Kombination von Matrix und Gel im zellfreien Transplantat der vorliegenden Erfindung hat auch den Vorteil, dass das Gel eine mechanische Barriere gegenüber anderen Zellen als mesenchymalen Vorläuferzellen des durch die Pridie-Bohrung oder ähnliche Frakturen eindringenden Blutes ausbildet. Hierdurch wird eine selektive Einwanderung der mesenchymalen Vorläuferzellen in das Transplantat ermöglicht. Nur diese setzen sich daher in der Matrix fest und differenzieren zu den gewünschten Gewebszellen aus. Eine Überwucherung der erwünschten gewebsbildenden Zellen durch andere Zellen erfolgt damit nicht oder läßt sich wesentlich verringern.

Gleichzeitig vermittelt das Gel durch seine Viskosität dem Implantat eine viskoelastische Eigenschaft, wodurch die mechanischen Eigenschaften des Implantates den Eigenschaften der natürlichen Biomatrix des Knorpels angeglichen werden. Dieses Angleichen der mechanischen Eigenschaften bzw. der Festigkeit des Implantates an das umliegenden Gewebe schont das umliegende und im Falle des Gelenks das gegenüberliegende Knorpelgewebe und ermöglicht eine frühere Belastung des Gelenks nach Erhalt des Implantates durch den Patienten. Des weiteren schützen die durch die Viskosität des Gels erzielten viskoelastischen Eigenschaften des Implantates das unterliegende Gewebe vor mechanischen Stoß- und Druckbelastungen, welches ein Heilen des Defektes unterstützt.

Da durch Trocknung der Feuchtigkeitsgehalt des Matrix-Gel-Transplantats vor der Implantation gezielt eingestellt werden kann, ist es möglich die Elastizität/Härte des Transplantats dem Patienten anzupassen, so dass dieser kein Fremdkörpergefühl nach der Implantation verspürt.

Zudem ermöglicht das erfindungsgemäße zellfreie Transplantat aufgrund der offenen Porosität der Matrix ein Eindringen der nicht-zellulären Komponenten des Blutes durch Diffusion, welches die effektive Gerinnung des Blutes und somit Blutstillung im Defektareal nach einer Mirkofrakturierung bzw. Pridie-Bohrung ermöglicht. Die Abdeckung des Defektes nach einer Mikrofrakturierung mit dem erfindungsgemäßen Implantat führt zur Blutstillung, welches die frühere Heilung des Defektes ermöglicht.

Das oben beschriebene zellfreie Transplantat kann hergestellt werden nach einem Verfahren, bei dem man die Matrix mit dem Gel in Kontakt bringt. Dieses In-Kontakt-Bringen kann durch Auftropfen, Einweichen, Imprägnieren und/oder Tränken erfolgen.

Das erfindungsgemäße Verfahren enthält einen Trocknungsschritt. Die Verwendung eines Trocknungsschrittes hat den Vorteil, daß das Transplantat in trockener Form länger lagerfähig ist. Falls das getrocknete zellfreie Transplantat vor der Verwendung zur Implantation mit einer wäßrigen Lösung, wie physiologische Kochsalzlösung und/oder synoviale Flüssigkeit kombiniert wird, kann dieses durch beispielsweise Tränken oder Einweichen erfolgen. Durch das erneute In-Kontakt-Bringen des getrockneten Transplantats mit einer wässrigen Lösung kann dann auch gleichzeitig die Elastizität/Härte des Transplantats individuell auf den Patienten angepasst werden.

Das Trocknen des zellfreien Transplantates kann durch Konvektionstrocknung, Lufttrocknung, Vakuumtrocknung, Kondensationstrocknung, Mikrowellentrocknung, Gefriertrocknung, Wärmetrocknung, chemische Trocknung, oder dielektrische Trocknung erfolgen. Vorzugsweise erfolgt die Trocknung durch Gefriertrocknung.

Für die oben genannte bevorzugte Ausführungsform aus Polyglycolsäuervlies mit Hyaluronsäuregel werden bei Vliesgrößen von 20 mm x 30 mm x 1,1 mm circa 600 µl einer Hyaluronsäurelösung (10 mg/ml) in physiologisch geeigneter Lösung in das Material eingebracht und mittels Gefriertrocknung getrocknet. Die trockenen zellfreien Implantate können durch Tränken mit 1 bis 2 ml Lösung befeuchtet werden. Vorzugsweise erfolgt das Tränken mit physiologischer Kochsalzlösung, mit synovialer Flüssigkeit und/oder mit verdünnter synovialer Flüssigkeit.

Das erfindungsgemäße zellfreie Matrix-Gel-Transplantat kann zur Abdeckung und Erhöhung der Viskoseelastizität von Defekten zur Gewebsregeneration von mesenchymalen Geweben und insbesondere zur Regeneration von Knorpel und/oder Knochen verwendet werden. Vorzugsweise wird es zur Regeneration mesenchymaler Gewebe verwendet. Am meisten bevorzugt ist die Verwendung zur Knorpelregeneration, insbesondere nach Pridie-Bohrung oder Mikrofrakturierung. Das Implantat fungiert als intelligente Abdeckung, welche nach einer Pridie-Bohrung oder Mikrofrakturierung zur Wiederherstellung der Gelenkoberfläche in den Knorpel passgenau eingebracht wird. Das Matrixmaterial, vorzugsweise Filzmaterial dient der mechanischen Stabilität und fungiert als Leitstruktur, welche die homogene, dreidimensionale Verteilung von aus dem Knochenmark bzw. spongiösem Knochen einwandernden Patientenzellen fördert und blutstillend wirkt. Das Gel wie beispielsweise Hyaluronsäure wirkt als Barriere, um die Einwanderung von roten Blutzellen und Leukozyten zu verhindern und vermittelt dem Implantat seine viskoelastischen Eigenschaften, welche das umliegende und unterliegende Gewebe vor mechanischer Beanspruchung schützt. Durch das Trockenen des Implantates wird eine längere Lagerfähigkeit erzielt und ein passives Eindringen von körpereigenen synovialen Flüssigkeiten oder Botenstoffen ermöglicht. Überraschend hat sich gezeigt, dass die Verwendung von synovialer Flüssigkeit deutlich gesteigerte Rekrutierungszahlen gegenüber der Verwendung von Wachstums- und Differenzierungsfaktoren sowie Chemokinen bzw. vergleichbare Rekrutierungszahlen wie Serum ermöglicht (siehe Figur 3).

Die folgenden Beispiele sollen die vorliegende Erfindung lediglich veranschaulichen, diese jedoch nicht beschränken.

### Beispiel 1:

Ein kommerziell im Handel unter der Marke PGA-Soft Felt® von der Alpha Research Switzerland GmbH vertriebenes Polyglykolsäurevlies wurde auf die Abmessungen von 20 mm x 30 mm x 1,1 mm geschnitten. Das Material wurde mit 0,6 ml kommerziell im Handel unter der Marke Ostenil® von der Firma TRB Chemedica AG vertriebener Hyaluronsäure einer Konzentration von 10 mg/ml mit Hilfe eines automatischen Perfusors getränkt. Die so erhaltene Matrix-Gel-Kombination wurde über circa 17 Stunden mit dem Gefriertrockner Epsilon 2-6 LSC getrocknet. Hierzu wurde die Gel-Matrix-Kombination in 90 Minuten von 20°C auf -20°C gekühlt und für 3 Stunden bei -20°C belassen. Als weiterer Trocknungsschritt wurde ein Vakuum von 1,03 mbar bei -20°C für 45 Minuten angelegt. Danach erfolgt das Aufheizen des Matrix-Gel-Transplantats von -20°C auf 20°C in 2 Stunden bei 1,03 mbar, um für weiterer 6,5 Stunden bei 20°C und 1,03 mbar zu trocknen. Im letzten Trocknungsschritt wird innerhalb 1 Stunde die Temperatur auf 25°C erhöht und der Druck auf 0,011 mbar erniedrigt. Nach weiteren 2 Stunden bei 25°C und 0,011 mbar ist der letzte Trocknungsschritt abgeschlossen.

Die Menge an verdrängter bzw. getrockneter Flüssigkeit im zellfreien Matrix-Gel-Transplantat wurde mittels Gewichtsbestimmung festgestellt. Das jeweilige bestimmte Gewicht ist in Figur 1 dargestellt. Im Mittel wogen 0,6 ml Hyaluronsäure 0,589 mg (HA). Soft PGA Felt® der Größe 20 x 30 x 1,1 mm wog im Mittel 0,155 mg (Matrix). Das Naßgewicht der Matrix-Gel-Kombination vor der Trocknung im Gefriertrocknung betrug im Mittel 0,744 mg (Nassgewicht HA + Matrix). Das Trockengewicht der Matrix-Gel-Kombination nach Gefriertrocknung betrug im Mittel 0,166 mg (Trockengewicht HA + Matrix). Das Gewicht der durch Trocknung aus der Matrix-Gel-Kombination verdrängten Flüssigkeit betrug im Mittel 0,579 mg (Verdrängte Flüssigkeit).

Nach Trocknung der Matrix-Gel-Kombination steht das zellfreie Transplantat zur Anwendung/oder Lagerung bereit.

### Beispiel 2:

Ein kommerziell im Handel unter der Marke PGA-Soft Felt® von der Alpha Research Switzerland GmbH vertriebenes Polyglykolsäurevlies wurde auf die Abmessungen von 20 mm x 15 mm x 1,1 mm geschnitten. Das Material wurde mit 0,3 ml kommerziell im Handel unter der Marke Ostenil® von der Firma TRB Chemedica AG vertriebener Hyaluronsäure einer Konzentration von 10 mg/ml getränkt. Das so erhaltene Matrix-Gel-Transplantat wurde für 17 Stunden im Gefriertrockner getrocknet.

Das Beibehalten von viskoelastischen Eigenschaften des zellfreien Matrix-Gel-Transplantates nach der Gefriertrocknung wurde mittels Messung der dynamischen Viskosität gezeigt. Die erhaltenen Viskostätsmeßwerte sind in der Figur 2 gezeigt. Zur Messung der dynamischen Viskosität wurde das trockene zellfreie Matrix-Gel-Transplantat mit 0,3 ml physiologischer Kochsalzlösung versetzt und für 16 Stunden bei 4°C unter leichtem Schütteln inkubiert. Zur Gewinnung der im Transplantat enthaltenen, rehydrierten Hyaluronsäure wurde das Transplantat in eine in einem Reaktionsgefäß stehende Pipettenspitze (1.000 µl) überführt und bei 2.000 rpm für 10 Minuten zentrifugiert. Die Messung der dynamischen Viskosität erfolgte in 1:10 Verdünnung mit physiologischer Kochsalzlösung im automatischen Microvikosimeter AMVn bei 20°C.

Zum Vergleich wurde die dynamische Viskosität von physiologischer Kochsalzlösung, von der Hyaluronsäure Ostenil® in 1:10 Verdünnung mit physiologischer Kochsalzlösung und von der Hyaluronsäure aus der Matrix-Gel-Kombination vor der Gefriertrocknung in 1:10 Verdünnung mit physiologischer Kochsalzlösung bestimmt. Für die physiologische Kochsalzlösung wurde im Mittel eine dynamische Viskosität von 1,09 mPa*s (phys. Kochsalzlsg.) und für die Hyaluronsäure Ostenil® in 1:10 Verdünnung mit physiologischer Kochsalzlösung von im Mittel 5,48 mPa*s (HA) bestimmt. Die dynamische Viskosität der Hyaluronsäure in der Matrix-Gel-Kombination vor der Trocknung im Gefriertrockner betrug im Mittel 5,54 mPa*s (HA + Matrix vor Trocknung). Nach der Gefriertrocknung betrug die dynamische Viskosität der Hyaluronsäure im zellfreien Transplantat im Mittel 5,69 mPa*s (HA + Matrix nach Trocknung). Dieses zeigt, daß im Herstellungsprozeß die viskoelastischen Eigenschaften der Hyaluronsäure nicht verändert werden.

Nach Trocknung des Matrix-Gel-Transplantats steht dieses zur Anwendung oder Lagerung bereit.

### Beispiel 3:

Ein Polyglykolsäurevlies mit den Abmessungen 20 mm x 30 mm x 1,1 mm wird mit 0,6 ml Hyaluronsäure einer Konzentration von 10 mg/ml getränkt. Die so erhaltene Matrix-Gel-Kombination wird, wie in Beispiel 1 dargelegt, im Gefriertrockner getrocknet.

Zur Anwendung wird das trockene zellfreie Matrix-Gel-Transplantat für 5 Minuten in physiologischer Kochsalzlösung inkubiert.

Ein Gelenkknorpeldefekt des Knies wird arthroskopisch gesäubert und mittels Mikrofrakturierung nach üblichem Verfahren behandelt. Das zellfreie Matrix-Gel-Transplantat wird in das Gelenk eingeführt und zur Abdeckung des mikrofrakturierten Defektes und zur Blutstillung genutzt. Das Fixieren der Abdeckung im Defekt kann durch Einkleben mit Fibrinkleber, durch Vernähen der Matrix mit dem umliegenden Gelenkknorpel (Knorpelnaht), durch Verankern der Matrix im subchondralen Knochen (transossäre Fixierung) oder durch Fixierung der Matrix im Defekt mittels im Knochen versenkter resorbierbarer Pins oder Nägel erfolgen.

### Beispiel 4:

Die Testung der chemotaktischen Wirkung von Synovialflüssigkeit von Normalspendern und Spendern mit Osteoarthrose auf mesenchymale Vorläuferzellen des Knochenmarks ergab überraschenderweise, daß humane Synovialflüssigkeit von gesunden Spendern und von Spendern mit Osteoarthrose im Vergleich zu Serum eine vergleichbare Zahl von Vorläuferzellen rekrutiert. Die Zellzahlen der im Mittel mit entsprechenden Standardabweichungen rekrutierten mesenchymalen Vorläuferzellen sind in Figur 3 dargestellt.

Die Verwendung von 10% fötalem bovinen Serum konnte im Mittel 11.143 Vorläuferzellen zur Wanderung in vitro anregen (10% FBS). 5% humanes Serum regte im Mittel 10.715 Vorläuferzellen zur Wanderung an (5% HS). Synovialflüssigkeit von Spendern mit Osteoarthrose regte in einer 1:2 Verdünnung mit dem Zellkulturmedium DMEM im Mittel 8.907 Zellen und Synovialflüssigkeit von normalen Spendern, ebenfalls in 1:2 Verdünnung in DMEM, regte im Mittel 9.920 Vorläuferzellen zur Wanderung an.

In der DE 10 2005 030 614 ist die Anzahl an mesenchymalen Stamm- und Vorläuferzellen, welche durch die Wachstums- und Differenzierungsfaktoren CDMP1 und CDMP2 rekrutiert wurden, mit maximal 156 bzw. maximal 38 Zellen angegeben. Des weiteren ist offenbart, dass das Chemokin SDF1-α maximal 79 und durch das Chemokin IL-8 maximal 814 Zellen pro 25 mm² zur Wanderung angeregt haben. Humanes Serum regte je nach Formulierung zwischen 2.135 und 10.332 mesenchymale Zellen zur Wanderung an.

### Beispiel 5:

Ein Polyglykolsäurevlies mit den Abmessungen 20 mm x 30 mm x 1,1 mm wird mit 0,6 ml Hyaluronsäure einer Konzentration von 10 mg/ml getränkt. Die so erhaltene Matrix-Gel-Transplantat wird, wie in Beispiel 1 beschrieben, im Gefriertrockner getrocknet.

Zur Anwendung wird das trockene zellfreie Transplantat für 10 Minuten in mit physiologischer Kochsalzlösung im Verhältnis 1:2 verdünnter, autologer Synovialflüssigkeit getränkt, welche dem zu behandelnden Patienten intraoperativ entnommen wurde.

Ein Gelenkknorpeldefekt des Knies wird arthroskopisch gesäubert und mittels Mikrofrakturierung nach üblichem Verfahren behandelt. Das in Synovialflüssigkeit getränkte zellfreie Transplantat wird in das Gelenk eingeführt und zur Abdeckung des mikrofrakturierten Defektes genutzt. Das Fixieren der Abdeckung im Defekt kann durch Einkleben mit Fibrinkleber, durch Vernähen der Matrix mit dem umliegenden Gelenkknorpel (Kuorpelnaht), durch Verankern der Matrix im subchondralen Knochen (transossäre Fixierung) oder durch Fixierung der Matrix im Defekt mittels im Knochen versenkter resorbierbarer Pins oder Nägel erfolgen.

## Patentansprüche

1. Zellfreies Transplantat, bestehend aus
(i) einer zusammenhängenden, gerüstbildenden Matrix mit offener Porosität aus einem biologisch und pharmazeutisch annehmbaren Material, wobei die Matrix ein Material umfasst, ausgewählt aus der Gruppe, bestehend aus Polyglycolsäure, Polymilchsäure, Poly(glycolid,lactat) und Gemischen davon, und
(ii) einem Gel aus einem biologisch und pharmazeutisch annehmbaren Material, wobei das Gel auf mindestens einer Seite der Matrix aufgebracht ist und/oder diese mindestens teilweise durchdringt und das Gel Hyaluronsäuregel ist.

2. Zellfreies Transplantat nach Anspruch 1, worin die Matrix resorbierbar ist.

3. Zellfreies Transplantat nach Anspruch 1 oder 2, worin die Matrix eine Struktur aufweist, ausgewählt unter Geweben oder Gewirken insbesondere Vlies- und Filzstrukturen, Membranen, Schwämmen, Watte, offenzelligen Schäumen, Wolle, Flechtwerk, geordneten und ungeordneten Faserbündeln, Spongiosa und Gelen sowie Kombinationen derselben.

4. Zellfreies Transplantat nach einem der vorstehenden Ansprüche, worin die Matrix ein Material umfasst, ausgewählt aus der Gruppe, bestehend aus Polyglycolsäure, Polymilchsäure und Gemischen davon.

5. Zellfreies Transplantat nach einem der vorstehenden Ansprüche, worin die Matrix Polyglycolsäure umfasst oder Polyglycolsäure ist.

6. Zellfreies Transplantat nach Anspruch 1, worin das Gel ein natürliches oder synthetisches Hydrogel ist.

7. Zellfreies Transplantat nach Anspruch 1 oder 6, worin das Gel auf die Matrix aufgetrocknet wurde.

8. Zellfreies Transplantat nach Anspruch 7, wobei die Auftrocknung des Gels durch Konvektionstrocknung, Lufttrocknung, Vakuumtrocknung, Kondensationstrocknung, Mikrowellentrocknung, Gefriertrocknung, Wärmetrocknung, chemische Trocknung, oder dielektrische Trocknung, und vorzugsweise durch Gefriertrocknung erfolgt.

9. Verfahren zur Herstellung eines zellfreien Transplantats nach einem der vorstehenden Ansprüche, umfassend die folgenden Schritte:
(v) In-Kontakt-bringen der Matrix mit dem Gel, wobei das Gel auf mindestens einer Seite der Matrix aufgebracht ist und/oder diese mindestens teilweise durchdringt und das Gel Hyaluronsäuregel ist, und wobei die Matrix ein Material umfasst, ausgewählt aus der Gruppe, bestehend aus Polyglycolsäure, Polymilchsäure, Poly(glycolid,lactat) und Gemischen davon, und
(vi) Trocknen des in (v) gebildeten Matrix-Gel-Komplexes.

10. Zellfreies Transplantat nach einem der Ansprüche 1 bis 8 zur Verwendung zur Abdeckung und Erhöhung der Viskoelastizität von Defekten.

11. Zellfreies Transplantat nach Anspruch 10 zur Verwendung zur Abdeckung von Defekten mesenchymaler Gewebe, insbesondere Knorpel und/oder Knochen.

12. Zellfreies Transplantat nach einem der Ansprüche 1 bis 8 zur Verwendung zur Gewebsregeneration.

13. Zellfreies Transplantat nach Anspruch 12 zur Verwendung zur Regeneration mesenchymaler Gewebe, insbesondere Knorpel und/oder Knochen.

## Claims

1. A cell-free graft consisting of
(i) a cohesive, scaffold-forming matrix with open porosity composed of a biologically and pharmaceutically acceptable material, wherein the matrix comprises a material selected from the group consisting of polyglycolic acid, polylactic acid, poly(glycolide, lactate) and mixtures thereof, and
(ii) a gel of a biologically and pharmaceutically acceptable material, wherein the gel is applied to at least one side of the matrix and/or at least partially penetrates the latter and the gel is hyaluronic acid gel.

2. The cell-free graft according to claim 1, in which the matrix is absorbable.

3. The cell-free graft according to claim 1 or 2, in which the matrix has a structure selected from wovens or knits, in particular nonwoven and felt structures, membranes, sponges, wadding, open-cell foams, wool, braids, ordered and unordered fiber bundles, spongiosa and gels, and combinations thereof.

4. The cell-free graft according to any one of the preceding claims, in which the matrix comprises a material selected from the group consisting of polyglycolic acid, polylactic acid and mixtures thereof.

5. The cell-free graft according to any one of the preceding claims, in which the matrix comprises polyglycolic acid or is polyglycolic acid.

6. The cell-free graft according to claim 1, in which the gel is a natural or synthetic hydrogel.

7. The cell-free graft according to claim 1 or 6, in which the gel has been dried onto the matrix.

8. The cell-free graft according to claim 7, wherein the drying of the gel takes place by convection drying, air drying, vacuum drying, condensation drying, microwave drying, freeze drying, heat drying, chemical drying, or dielectric drying, and preferably by freeze drying.

9. A method for producing a cell-free graft according to any one of the preceding claims, comprising the following steps:
(v) contacting the matrix with the gel, wherein the gel is applied to at least one side of the matrix and/or at least partially penetrates the latter and the gel is hyaluronic acid gel, and wherein the matrix comprises a material selected from the group consisting of polyglycolic acid, poly lactic acid, poly(glycolide, lactate) and mixtures thereof, and
(vi) drying the matrix-gel complex formed in (v).

10. The cell-free graft according to any one of claims 1 to 8 for the use of covering and increasing the viscoelasticity of defects.

11. The cell-free graft according to claim 10 for the use of covering defects of mesenchymal tissue, especially cartilage and/or bone.

12. The cell-free graft according to any one of claims 1 to 8 for the use of tissue regeneration.

13. The cell-free graft according to claim 12 for the use in regenerating mesenchymal tissue, especially cartilage and/or bone.

## Revendications

1. Greffon acellulaire, qui consiste en
(i) une matrice cohésive, formant support avec une porosité ouverte, qui consiste en une matière acceptable au point de vue biologique et pharmaceutique, sachant que la matrice comprend une matière, choisie dans le groupe comprenant l'acide polyglycolique, l'acide polylactique, le polyglycolide, le polylactate et des mélanges de ceux-ci, et
(ii) un gel à base d'une matière acceptable au point de vue biologique et pharmaceutique, sachant que le gel est appliqué sur au moins une face de la matrice et / ou pénètre au moins partiellement celle-ci, et que ledit gel est un gel d'acide hyaluronique.

2. Greffon acellulaire selon la revendication 1, dans lequel la matrice peut être résorbée.

3. Greffon acellulaire selon revendication 1 ou 2, dans lequel la matrice est dotée d'une structure choisie parmi des tissus ou des tissus à mailles, en particulier des structures de non tissé et de feutre, de membranes, de l'éponge, de la ouate, des mousses à cellules ouvertes, de la laine, des tressés, des faisceaux de fibres ordonnés ou désordonnés, des spongieux et des gels, ainsi que des combinaisons de ceux-ci.

4. Greffon acellulaire selon l'une des revendications précédentes, dans lequel la matrice comprend une matière choisie dans le groupe comprenant l'acide polyglycolique, l'acide polylactique et des mélanges de ceux-ci.

5. Greffon acellulaire selon l'une des revendications précédentes, dans lequel la matrice comprend de l'acide polyglycolique ou est de l'acide polyglycolique.

6. Greffon acellulaire selon la revendication 1, dans lequel le gel est un hydrogel naturel ou synthétique.

7. Greffon acellulaire selon revendication 1 ou 6, dans lequel le gel a été séché sur la matrice.

8. Greffon acellulaire selon la revendication 7, sachant que le séchage du gel est effectué par séchage par convection, séchage à l'air, séchage sous vide, séchage par condensation, séchage par micro-ondes, séchage par lyophilisation, séchage par la chaleur ou séchage diélectrique, et, de préférence, par lyophilisation.

9. Procédé de fabrication d'un greffon acellulaire selon l'une des revendications précédentes, qui comprend les étapes suivantes :
(v) Mise en contact de la matrice avec le gel, sachant que le gel est appliqué sur au moins une face de la matrice et / ou pénètre au moins partiellement celle-ci, et que ledit gel est du gel d'acide hyaluronique, et que la matrice comprend une matière choisie dans le groupe comprenant l'acide polyglycolique, l'acide polylactique, le polyglycolide et le polylactate et des mélanges de ceux-ci,
(vi) Séchage du complexe matrice et gel, formé sous (v).

10. Greffon acellulaire selon l'une des revendications 1 à 8, destiné à être utilisé pour couvrir et augmenter la visco-élasticité d'anomalies.

11. Greffon acellulaire selon la revendication 10, destiné à être utilisé pour couvrir des anomalies de tissus mésenchymateux, en particulier de cartilage et /ou d'os.

12. Greffon acellulaire selon l'une des revendications 1 à 8, destiné à être utilisé pour la régénération de tissus.

13. Greffon acellulaire selon la revendication 12, destiné à être utilisé pour la régénération de tissus mésenchymateux, en particulier de cartilage et / ou d'os.
